# EUROPEAN PATENT APPLICATION

(11) **EP 4 534 033 A1**
(43) Date of publication of application: **09.04.2025**
(21) Application number: 22945030.9
(22) Date of filing: 17.11.2022
(51) Int. Cl.: A61B 34/10, A61B 90/00

(54) **METHOD, DEVICE, AND PROGRAM FOR PROVIDING PATIENT-SPECIFIC 3D SURGICAL SIMULATION**

(30) Priority: 30.05.2022 KR 20220066184
(71) Applicant: Hutom Inc., Seoul 04151 (KR)
(72) Inventor: KIM, Sung Jae, Seoul 03453 (KR); HAN, Yejin, Seoul 06624 (KR)
(74) Representative: dompatent von Kreisler Selting Werner - Partnerschaft von Patent- und Rechtsanwälten mbB
(86) International application number: PCT/KR2022/018194
(87) International publication number: WO 2023/234492

(57) **Abstract**

A method for providing a patient-specific 3D surgical simulation method, according to the present invention, may comprise the steps of: acquiring a scan image including an image of a target object outputted by scanning a patient's body; identifying images of the target object and one or more organs and blood vessels adjacent to the target object in the scan image; generating a first 3D model of a surgical area on the basis of first 3D modeling data of the identified image and second 3D modeling data related to the one or more organs and blood vessels; setting third 3D modeling data and environmental variables for surgical equipment to be applied to the first 3D model; generating a second 3D model for the surgical equipment on the basis of the third 3D modeling data and the environmental variables; and providing a patient-specific surgical simulation on the basis of the first 3D model and the second 3D model.

## Description

### [TECHNICAL FIELD]

The present disclosure relates to a method, an apparatus, and a program for providing a simulation. More particularly, the present disclosure relates to a method, an apparatus, and a program for providing a patient-specific 3D surgical simulation.

### [BACKGROUND ART]

Recently, in the case of surgery in hospitals, before performing the surgery, a patient's condition and conditions for performing the surgery are checked, and in order to increase the probability of the success of the surgery, a 3D simulation (stereoscopic image) of the patient's surgical site is created, and the surgery is performed virtually under the same conditions as the actual surgery.

In the case of such a virtual simulation surgery, a precise diagnosis of the patient's body can be made and a surgical plan can be established in advance. Accordingly, by performing a virtual simulation surgery rather than relying on the intuition of a specialist, even very small errors related to the surgery can be reduced.

However, the virtual simulation surgery has the problem of lacking realism. In addition, in the case of surgical surgery, there is a problem that the medical staff cannot perform a virtual simulation surgery under the same conditions as the actual surgery.

### [DETAILED DESCRIPTION OF THE INVENTION]

### [TECHINICAL PROBLEM]

The present disclosure is to provide a method, device, and program for providing a patient-specific 3D surgical simulation.

Technical problems of the inventive concept are not limited to the technical problems mentioned above, and other technical problems not mentioned will be clearly understood by those skilled in the art from the following description.

### [TECHNICAL SOLUTION]

In an aspect of the present disclosure, a method for providing a patient-specific 3D surgical simulation method may include obtaining a scan image including an image of a target object output by scanning a patient's body; identifying an image of the target object and one or more organs and blood vessels adjacent to the target object on the scan image; generating a first 3D model for a surgical area based on first 3D modeling data for the identified image and separate second 3D modeling data related to the one or more organs and blood vessels; configuring third 3D modeling data and an environmental variable for a surgical equipment to be applied to the first 3D model; generating a second 3D model for the surgical equipment based on the third 3D modeling data and the environmental variable; and providing the patient-specific 3D surgical simulation based on the first 3D model and the second 3D model.

Furthermore, the scan image may include a computer tomography (CT) image of the patient's body, and identifying the image may include detecting and configuring a boundary of each image of a plurality of objects included in the CT image; segmenting the image of the plurality of objects according to the configured boundary; and identifying an image of the target object and an image of the organ and the blood vessel adjacent to the target object among the images of the segmented plurality of objects.

Furthermore, the second 3D modeling data may include at least one of data modeled in advance in 3D for each of the one or more organs and blood vessels, or data modeled in 3D for each of the one or more organs and blood vessels acquired through an artificial intelligence (AI) model.

Furthermore, generating the first 3D model may include: comparing fourth 3D modeling data for the one or more organs and blood vessels included in the first 3D modeling data with the second 3D modeling data; and combining the fourth 3D modeling data and the second 3D modeling data based on the comparison result.

Furthermore, the comparison result may include 3D modeling data for a specific portion of the one or more organs and blood vessels included in the second 3D modeling data that is not included in the CT image, and generating the first 3D model may include combining the 3D modeling data for the specific portion with the fourth 3D modeling data.

Furthermore, combining the 3D modeling data may include applying at least one of a smoothing filter or a blending filter to the boundary portion of the 3D modeling data for the specific portion and the fourth 3D modeling data.

Furthermore, generating the first 3D model may include: uploading fifth 3D modeling data for a body tissue within a preconfigured distance from the target object; and generating the first 3D model based on the first 3D modeling data, the second 3D modeling data, or the fifth 3D modeling data, and the fifth 3D modeling data is template data on which a rigging operation is performed so that other 3D modeling data is capable of being combined.

Furthermore, the environmental variable may include at least one of pivot-related configuration information for a movement of the surgical equipment or configuration information for an effect that the surgical equipment is capable of applying to the first 3D model.

In another aspect of the present disclosure, a device for providing a patient-specific 3D surgical simulation may include one or more communication modules; one or more memories; and one or more processors, wherein the one or more processors are configured to: obtain a scan image including an image of a target object output by scanning a patient's body; identify an image of the target object and one or more organs and blood vessels adjacent to the target object on the scan image; generate a first 3D model for a surgical area based on first 3D modeling data for the identified image and separate second 3D modeling data related to the one or more organs and blood vessels; configure third 3D modeling data and an environmental variable for a surgical equipment to be applied to the first 3D model; generate a second 3D model for the surgical equipment based on the third 3D modeling data and the environmental variable; and provide the patient-specific 3D surgical simulation based on the first 3D model and the second 3D model.

Furthermore, a computer program stored in a computer-readable recording medium for executing the present disclosure may be further provided.

Furthermore, a computer-readable recording medium recording a computer program for executing a method for executing the present disclosure may be further provided.

### [ADVANTAGEOUS EFFECTS OF THE INVENTION]

According to the above-described problem solving means of the present disclosure, a method, device, and program for providing a patient-specific 3D surgical simulation can be provided.

In addition, according to the above-described problem solving means of the present disclosure, it is possible to confirm the human body structure of an actual patient in a 3D simulation, and it can be helpful for actual surgery.

The effects of the present disclosure are not limited to the effects mentioned above, and other effects not mentioned can be clearly understood by those skilled in the art from the description.

### [DESCRIPTION OF THE DRAWINGS]

FIG. 1 is a schematic diagram of a system for implementing a method for providing patient-specific 3D surgical simulation according to an embodiment of the present disclosure.
FIG. 2 is a block diagram illustrating a configuration of the device for providing a patient-specific 3D surgical simulation according to an embodiment of the present disclosure.
FIG. 3 is a flowchart for describing a method for providing a patient-specific 3D surgical simulation through a device according to one embodiment of the present disclosure.
FIG. 4 is a flowchart for describing a method for generating a first 3D model for a surgical area according to an embodiment of the present disclosure.
FIG. 5 is a diagram for describing a patient-specific 3D surgical simulation according to an embodiment of the present disclosure.

### [BEST MODE]

In the drawings, the same reference numeral refers to the same element. This disclosure does not describe all elements of embodiments, and general contents in the technical field to which the present disclosure belongs or repeated contents of the embodiments will be omitted. The terms, such as "unit, module, member, and block" may be embodied as hardware or software, and a plurality of "units, modules, members, and blocks" may be implemented as one element, or a unit, a module, a member, or a block may include a plurality of elements.

Throughout this specification, when a part is referred to as being "connected" to another part, this includes "direct connection" and "indirect connection", and the indirect connection may include connection via a wireless communication network. Furthermore, when a certain part "includes" a certain element, other elements are not excluded unless explicitly described otherwise, and other elements may in fact be included.

Furthermore, when a certain part "includes" a certain element, other elements are not excluded unless explicitly described otherwise, and other elements may in fact be included.

In the entire specification of the present disclosure, when any member is located "on" another member, this includes a case in which still another member is present between both members as well as a case in which one member is in contact with another member.

The terms "first," "second," and the like are just to distinguish an element from any other element, and elements are not limited by the terms.

The singular form of the elements may be understood into the plural form unless otherwise specifically stated in the context.

Identification codes in each operation are used not for describing the order of the operations but for convenience of description, and the operations may be implemented differently from the order described unless there is a specific order explicitly described in the context.

Unless otherwise defined, all terms (including technical and scientific terms) used in this specification may be used with a meaning that can be commonly understood by those skilled in the art to which the present invention belongs. In addition, terms defined in commonly used dictionaries shall not be ideally or excessively interpreted unless explicitly specifically defined.

Hereinafter, operation principles and embodiments of the present disclosure will be described with reference to the accompanying drawings.

In describing the present disclosure, an "image" may mean multidimensional data composed of discrete image elements (e.g., pixels in a 2D image and voxels in a 3D image). For example, the image may include a medical image of a subject obtained by a CT scan device, and the like.

In describing the present disclosure, an "object" may be a person (e.g., a patient) or an animal, or a part or all of a person or an animal. For example, the object may include at least one of an organ such as a liver, a heart, a uterus, a brain, a breast, an abdomen, and the like., and a blood vessel (e.g., an artery or a vein), adipose tissue, and the like. In addition, a "target object" may mean a part of a person that is the target of an actual surgery.

In describing the present disclosure, a "user" may be a medical professional such as a doctor, nurse, clinical pathologist, medical imaging specialist, etc., and may be a technician who repairs medical devices, but is not limited thereto.

In describing the present disclosure, "3D modeling data" means data that visualizes a specific object in 3D, and "3D model" may mean an element of a simulation generated by combining one or more 3D modeling data.

The "3D modeling data" or/and "3D model" may be provided in 2D on a display provided to a user, but may be provided so as to be displayed in a 3D form, or may be provided so as to be displayed so as to make a body part appear in real space through the display, such as augmented reality.

In describing the present disclosure, a "device" (i.e., a device that performs a patient-specific 3D surgical simulation method) includes all various devices that can perform computational processing and provide results to a user.

For example, the device may be a desktop PC, a notebook, a smart phone, a tablet PC, a cellular phone, a PCS phone (Personal Communication Service phone), a synchronous/asynchronous IMT-2000 (International Mobile Telecommunication-2000) mobile terminal, a Palm Personal Computer, a PDA (Personal Digital Assistant), and the like. In addition, in the case that a head mounted display (HMD) device includes a computing function, the HMD device may be the device. In addition, the device may be implemented as a separate server that receives a request from a client and performs information processing.

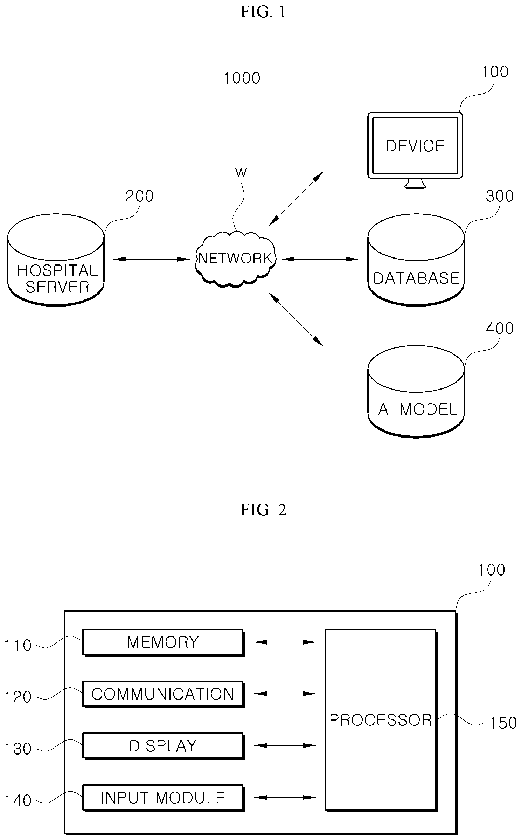

FIG. 1 is a schematic diagram of a system 1000 for implementing a method for providing patient-specific 3D surgical simulation according to an embodiment of the present disclosure.

As illustrated in FIG. 1, a system 1000 for implementing a method for providing patient-specific 3D surgical simulation may include a device 100, a hospital server 200, a database 300, and an AI model 400.

Here, although FIG. 1 illustrates that the device 100 is implemented in the form of a single desktop, it is not limited thereto. The device 100 may mean various types of devices or a group of devices in which one or more types of devices are connected, as described above.

The device 100, the hospital server 200, the database 300, and the AI (artificial intelligence) model 400 included in the system 1000 may perform communication via the network W. Here, the network W may include a wired network and a wireless network. For example, the network may include various networks such as a local area network (LAN), a metropolitan area network (MAN), and a wide area network (WAN).

In addition, the network W may include the well-known World Wide Web (WWW). However, the network W according to the embodiment of the present disclosure is not limited to the networks listed above, and may include at least a part of a well-known wireless data network, a well-known telephone network, and a well-known wired and wireless television network.

The device 100 may provide a patient-specific 3D surgical simulation method. For example, the device 100 may generate a 3D model for a surgical area based on a scan image including an image of a target object output by scanning a patient's body. The device 100 may generate a second 3D model related to surgical equipment to be applied to the first 3D model. The device 100 may provide a patient-specific surgical simulation based on the first 3D model and the second 3D model. The related operations will be specifically described with reference to the drawings described below.

The hospital server 200 (e.g., a cloud server, etc.) may store a scan image (e.g., a computer tomography (CT) image) obtained by scanning a patient's body. The hospital server 200 may transmit the scan image stored to the device 100, the database 300, or the AI model 400.

The hospital server 200 may protect personal information of the body by pseudonymizing or anonymizing the subject of the CT image. In addition, the hospital server may encrypt and store information related to the age/gender/height/weight/childbirth status of the patient who is the subject of the CT image input by a user.

The database 300 may store 3D modeling data and 3D models for various objects/surgical equipment generated by the device 100. As another example, the database 300 may store template data on which a rigging operation is performed so that it may be combined with various 3D modeling data. Although FIG. 1 illustrates a case where the database 300 is implemented outside the device 100, the database 300 may also be implemented as a component of the device 100.

The AI model 400 is an artificial intelligence model that is trained to output 3D modeling data for a specific object. The AI model 400 may be trained to output 3D modeling data for a specific object through a data set constructed with actual surgical images or CT images and anatomical data. The learning method may include, but is not limited to, supervised training/unsupervised training. The 3D modeling data for multiple objects output through the AI model 400 may be stored in the database 300 or/and the memory of the device 100.

FIG. 1 illustrates a case where the AI model 400 is implemented outside the device 100 (e.g., implemented in a cloud-based manner), but is not limited thereto, and may be implemented as a component of the device 100.

FIG. 2 is a block diagram illustrating a configuration of the device 100 for providing a patient-specific 3D surgical simulation according to an embodiment of the present disclosure.

As shown in FIG. 2, the device 100 may include a memory 110, a communication module 120, a display 130, an input module 140, and a processor 150. However, the present disclosure is not limited thereto, and the device 100 may have software and hardware configurations modified/added/omitted within a range obvious to those skilled in the art according to a required operation.

The memory 110 may store data supporting various functions of the device 100 and a program for the operation of the processor 150, may store input/output data (e.g., music files, still images, moving images, etc.), and may store a plurality of application programs or applications run on the device, data for the operation of the device 100, and commands. At least some of these application programs may be downloaded from an external server via wireless communication.

The memory 110 may include at least one type of storage medium among a flash memory type, a hard disk type, an SSD type (Solid State Disk type), an SDD type (Silicon Disk Drive type), a multimedia card micro type, a card type memory (e.g., an SD or XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk.

In addition, the memory 110 may include a database that is separate from the device but connected by wire or wirelessly. That is, the database illustrated in FIG. 1 may be implemented as a component of the memory 110.

The communication module 120 may include one or more components that enable communication with an external device, and may include, for example, at least one of a broadcast reception module, a wired communication module, a wireless communication module, a short-range communication module, or a location information module.

The wired communication module may include various wired communication modules such as a Local Area Network (LAN) module, a Wide Area Network (WAN) module, or a Value Added Network (VAN) module, as well as various cable communication modules such as a Universal Serial Bus (USB), a High Definition Multimedia Interface (HDMI), a Digital Visual Interface (DVI), RS-232 (recommended standard 232), power line communication, or a plain old telephone service (POTS).

The wireless communication module may include a wireless communication module that supports various wireless communication methods such as a WiFi module, a WiBro (Wireless broadband) module, GSM (Global System for Mobile Communication), CDMA (Code Division Multiple Access), WCDMA (Wideband Code Division Multiple Access), UMTS (Universal Mobile Telecommunications System), TDMA (Time Division Multiple Access), LTE (Long Term Evolution), 4G, 5G, and 6G.

The display 130 displays (outputs) information processed in the device 100 (e.g., 3D modeling data/model or a simulation screen based on 3D modeling data/model). For example, the display may display execution screen information of an application program (e.g., an application) running in the device 100, or UI (User Interface) or GUI (Graphical User Interface) information according to the execution screen information.

The input module 140 is for receiving information from a user. When information is input through the user input unit, the processor 150 may control the operation of the device 100 to correspond to the input information.

The input module 140 may include a hardware-type physical key (e.g., a button located on at least one of the front, rear, or side of the device, a dome switch, a jog wheel, a jog switch, etc.) and a software-type touch key. As an example, the touch key may be formed as a virtual key, a soft key, or a visual key displayed on a touchscreen-type display 130 through software processing, or as a touch key placed on a part other than the touchscreen. Meanwhile, the virtual key may be displayed on the touchscreen in various forms, and may be formed as, for example, a graphic, a text, an icon, a video, or a combination thereof.

The processor 150 may control the overall operation and function of the device 100. Specifically, the processor 150 may be implemented as a memory storing data for an algorithm for controlling the operation of components within the device 100 or a program reproducing the algorithm, and at least one processor (not shown) that performs the operation described above using the data stored in the memory. At this time, the memory and the processor may be implemented as separate chips, respectively. Alternatively, the memory and the processor may be implemented as a single chip.

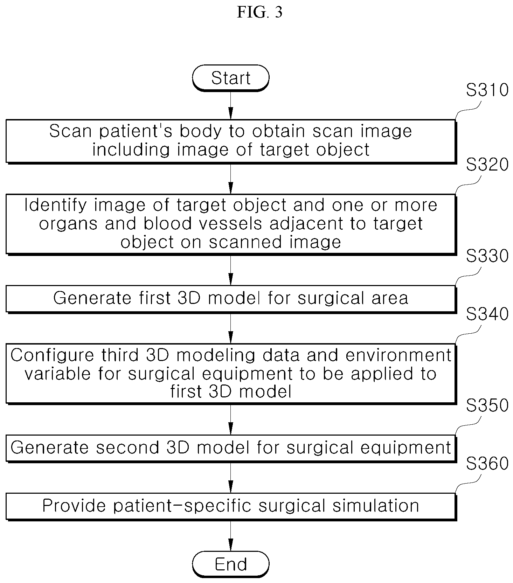

In addition, the processor 150 may control one or a combination of the components described above to implement various embodiments according to the present disclosure described in FIGS. 3 to 5 below on the device 100.

FIG. 3 is a flowchart for describing a method for providing a patient-specific 3D surgical simulation through a device according to one embodiment of the present disclosure.

The device may scan a patient's body to obtain a scan image including an image of a target object (step S310).

For example, the device may receive a CT image of a patient's body scanned from a hospital server. Specifically, the device may transmit a signal requesting a CT image of a specific patient containing an image of a target object to the hospital server or a device controlled by the hospital. The hospital server may transmit the CT image of a specific patient containing the image of the target object to the device according to the request signal of the device.

However, this is merely an embodiment, and the device may receive a CT image containing the image of the target object from a CT imaging device connected to the device wirelessly/wirelessly.

The device may identify the image of the target object and one or more organs and blood vessels adjacent to the target object on the scanned image (step S320).

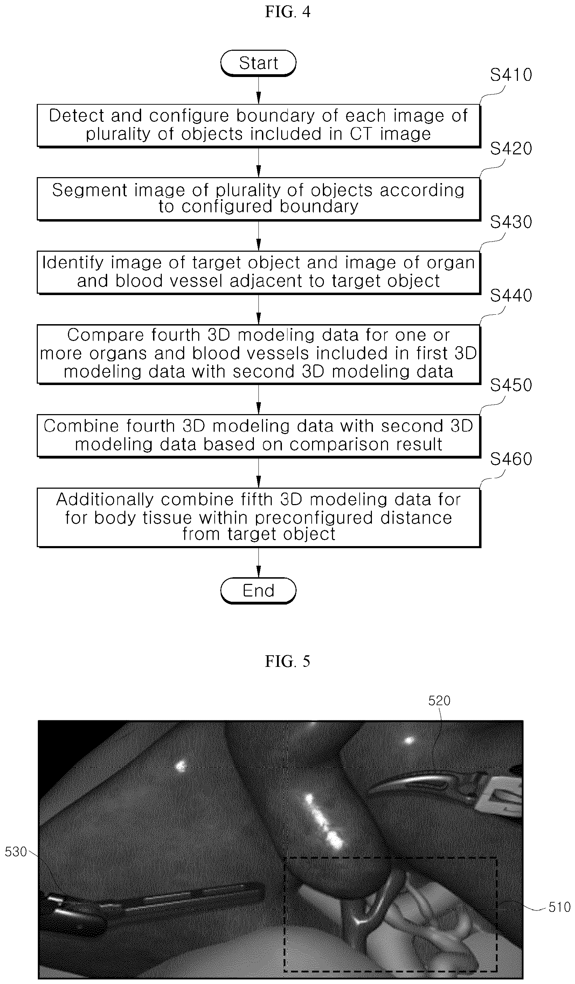

In addition, the device may generate a first 3D model for a surgical area (i.e., a predetermined area containing the target object) (step S330). The description of steps S320 and S330 will be specifically described with reference to FIG. 4.

The device may configure third 3D modeling data and an environment variable for the surgical equipment to be applied to the first 3D model (step S340). That is, the device may configure 3D modeling data and the environment variable for the surgical equipment to perform a virtual simulation surgery for the 3D model related to each object in the simulation.

Here, the environment variable may include at least one of pivot-related configuration information for the movement of the surgical equipment or configuration information for an effect that the surgical equipment may apply to the first 3D model.

For example, in the case that the specific surgical equipment is a scalpel, the environment variable of the scalpel may include pivot-related configuration information for the movement of the scalpel or configuration information for an effect that may be applied to the target object (e.g., an effect of cutting and slicing the target object).

The device may generate a second 3D model for the surgical equipment based on the third 3D modeling data and the environment variable (step S350).

The device may provide a patient-specific surgical simulation based on the first 3D model and the second 3D model (step S360).

That is, the device may generate/provide a (patient-specific) surgical simulation for a virtual mock surgery to the user by using the first 3D model based on 3D modeling data for each of the target object and the device/blood vessel located adjacent to or around the target object, and the second 3D model for the surgical equipment.

FIG. 4 is a flowchart for describing a method for generating a first 3D model for a surgical area according to an embodiment of the present disclosure. That is, FIG. 4 is a specific example of steps S320 to S330 described with reference to FIG. 3.

The device may detect and configure the boundary of each image of a plurality of objects included in the CT image (step S410). In addition, the device may segment the image of the plurality of objects according to the configured boundary (step S420). That is, the device may automatically detect and configure the boundary of each object to distinguish/segment the object included in the CT image.

In addition, the device may identify the image of the target object and the image of the organ and blood vessel adjacent to the target object among the images of the segmented multiple objects (step S430).

For example, assume that the target object is the gallbladder (i.e., assume that the surgery to be performed on the patient is a gallbladder removal surgery). The CT image acquired by the device may include the image of the gallbladder and the blood vessels/organs/fat tissue adjacent to the gallbladder. The device may detect and configure the boundary of each image of the gallbladder and the blood vessels/organs/fat tissue adjacent to the gallbladder. And the device may segment the image of the gallbladder and the blood vessels/organs/fat tissue adjacent to the gallbladder according to the detected and configure boundary. In addition, the device may identify the image of the target object and the image of the gallbladder and the blood vessels/organs/fat tissue adjacent to the gallbladder on the segmented image.

The image of the target object and the organs/blood vessels adjacent to the target object automatically segmented/detected/identified by the device may be inspected by other users. The device may modify the segmented/detected/identified image based on the user's inspection result.

The device may compare fourth 3D modeling data for one or more organs and blood vessels included in the first 3D modeling data with the second 3D modeling data (step S450). Then, the device may combine the fourth 3D modeling data with the second 3D modeling data based on the comparison result (step S460).

Here, the second 3D modeling data may include at least one of data modeled in advance in 3D for each of the one or more organs and blood vessels or the data 3D modeled for each of the one or more organs and blood vessels obtained through an AI model.

Then, the comparison result may include the 3D modeling data for a specific portion of the one or more organs and blood vessels included in the second 3D modeling data that is not included in the CT image. In other words, the specific portion may mean a portion of the image of the organ/blood vessel adjacent to the target object that is not included in the CT image.

The device may combine the 3D modeling data for the specific portion with the fourth 3D modeling data. That is, the device may generate modeling data for the organs/blood vessels adjacent to the target object completely by combining modeling data for the specific portion that is not included in the CT image with the fourth 3D modeling data.

When combining modeling data for the specific portion with the fourth 3D modeling data, at least one of a smoothing filter or a blending filter may be used. For example, the device may apply at least one of the smoothing filter or the blending filter to the boundary portion of the 3D modeling data for the specific portion and the fourth 3D modeling data. Accordingly, the boundary portion of the 3D modeling data for the specific portion and the fourth 3D modeling data may be expressed seamlessly.

The device may additionally combine fifth 3D modeling data for a body tissue within a preconfigured distance from the target object (step S470).

Specifically, the device may upload the fifth 3D modeling data for the body tissue within the preconfigured distance from the target object from a separate database. The fifth 3D modeling data may mean template data on which a rigging operation is performed so that it may be combined with other 3D modeling data.

Here, the body tissue within the preconfigured distance from the target object may mean the object located in an area surrounding the target object. For example, in the case that the target object is a gallbladder, the body tissue within the preconfigured distance may include an omentum formed over the intestines over the gallbladder, and the like.

The 3D modeling data for the omentum may be produced in a form that extends from above and covers the intestines. At this time, in order to connect the extending portion in the 3D modeling data for the target object generated based on the CT image, the rigging operation may be performed on top of the 3D modeling data for the omentum so that it may move with a templated operation. Accordingly, the stretched portion may be changed to correspond to different upper shapes, and more natural omentum 3D modeling data may be implemented.

The device may generate the first 3D model based on the first 3D modeling data, the second 3D modeling data, and the fifth 3D modeling data.

FIG. 5 is a diagram for describing a patient-specific 3D surgical simulation according to an embodiment of the present disclosure.

FIG. 5 illustrates an example screen of a simulation generated by the device. The device may generate the first 3D model 510 based on 3D modeling data for the target object and the blood vessels/organs adjacent to the target object. Through the simulation screen, the user may check the blood vessel structure and the organ shape in advance before performing a surgery.

In addition, the device may implement the second 3D model 520 and 530 for surgical equipment applicable to the first 3D model 510 on the simulation. The user may perform a simulated virtual surgery by applying the second model 520, 530 to the first 3D model 510.

Meanwhile, the disclosed embodiments may be implemented in the form of a recording medium storing instructions executable by a computer. The instructions may be stored in the form of program codes, and when executed by a processor, may generate a program module to perform the operations of the disclosed embodiments. The recording medium may be implemented as a computer-readable recording medium.

The computer-readable recording medium includes all types of recording media storing instructions that may be decoded by a computer. For example, there may be a ROM Read Only Memory, a RAM Random Access Memory, a magnetic tape, a magnetic disk, a flash memory, an optical data storage device, etc.

As described above, the disclosed embodiments have been described with reference to the attached drawings. A person skilled in the art will appreciate that the present disclosure may be practiced in other forms than the disclosed embodiments without changing the technical spirit or essential features of the present disclosure. The disclosed embodiments are illustrative and should not be construed as limiting.

## Claims

1. A method for providing a patient-specific 3D surgical simulation method performed by a device, comprising:
obtaining a scan image including an image of a target object output by scanning a patient's body;
identifying an image of the target object and one or more organs and blood vessels adjacent to the target object on the scan image;
generating a first 3D model for a surgical area based on first 3D modeling data for the identified image and separate second 3D modeling data related to the one or more organs and blood vessels;
configuring third 3D modeling data and an environmental variable for a surgical equipment to be applied to the first 3D model;
generating a second 3D model for the surgical equipment based on the third 3D modeling data and the environmental variable; and
providing the patient-specific 3D surgical simulation based on the first 3D model and the second 3D model.

2. The method according to claim 1,
wherein the scan image includes a computer tomography (CT) image of the patient's body, and
identifying the image includes:
detecting and configuring a boundary of each image of a plurality of objects included in the CT image;
segmenting the image of the plurality of objects according to the configured boundary; and
identifying an image of the target object and an image of the organ and the blood vessel adjacent to the target object among the images of the segmented plurality of objects.

3. The method according to claim 2,
wherein the second 3D modeling data includes at least one of data modeled in advance in 3D for each of the one or more organs and blood vessels, or data modeled in 3D for each of the one or more organs and blood vessels acquired through an artificial intelligence (AI) model.

4. The method according to claim 3,
generating the first 3D model includes:
comparing fourth 3D modeling data for the one or more organs and blood vessels included in the first 3D modeling data with the second 3D modeling data; and
combining the fourth 3D modeling data and the second 3D modeling data based on the comparison result.

5. The method according to claim 4,
wherein the comparison result includes:
3D modeling data for a specific portion of the one or more organs and blood vessels included in the second 3D modeling data that is not included in the CT image, and
wherein generating the first 3D model includes:
combining the 3D modeling data for the specific portion with the fourth 3D modeling data.

6. The method according to claim 5,
wherein combining the 3D modeling data includes:
applying at least one of a smoothing filter or a blending filter to the boundary portion of the 3D modeling data for the specific portion and the fourth 3D modeling data.

7. The method according to claim 6,
wherein generating the first 3D model includes:
uploading fifth 3D modeling data for a body tissue within a preconfigured distance from the target object; and
generating the first 3D model based on the first 3D modeling data, the second 3D modeling data, or the fifth 3D modeling data, and
wherein the fifth 3D modeling data is template data on which a rigging operation is performed so that other 3D modeling data is capable of being combined.

8. The method according to claim 7,
wherein the environmental variable includes at least one of pivot-related configuration information for a movement of the surgical equipment or configuration information for an effect that the surgical equipment is capable of applying to the first 3D model.

9. A device for providing a patient-specific 3D surgical simulation, comprising:
one or more communication modules;
one or more memories; and
one or more processors,
wherein the one or more processors are configured to:
obtain a scan image including an image of a target object output by scanning a patient's body;
identify an image of the target object and one or more organs and blood vessels adjacent to the target object on the scan image;
generate a first 3D model for a surgical area based on first 3D modeling data for the identified image and separate second 3D modeling data related to the one or more organs and blood vessels;
configure third 3D modeling data and an environmental variable for a surgical equipment to be applied to the first 3D model;
generate a second 3D model for the surgical equipment based on the third 3D modeling data and the environmental variable; and
provide the patient-specific 3D surgical simulation based on the first 3D model and the second 3D model.

10. A computer program stored in a computer-readable recording medium, coupled with a hardware device, for executing the method for providing a patient-specific 3D surgical simulation according to claim 1.
